# EUROPEAN PATENT APPLICATION

(11) **EP 4 614 514 A1**
(43) Date of publication of application: **10.09.2025**
(21) Application number: 22964544.5
(22) Date of filing: 04.11.2022
(51) Int. Cl.: G16H 50/20, G16H 50/50, G16H 50/70, G01N 21/65, G06N 3/08

(54) **ARTIFICIAL INTELLIGENCE-BASED MENTAL ILLNESS DIAGNOSIS SYSTEM AND METHOD USING EXOSOME SERS SIGNALS**

(30) Priority: 31.10.2022 KR 20220142034
(71) Applicant: Exopert Corporation, Seoul 02580 (KR); Korea University Research and Business Foundation, Seoul 02841 (KR)
(72) Inventor: SHIN, Hyunku, Seoul 02776 (KR); CHOI, Yeonho, Seoul 06276 (KR); HAM, Byung Joo, Seoul 04632 (KR)
(74) Representative: Mewburn Ellis LLP
(86) International application number: PCT/KR2022/017185
(87) International publication number: WO 2024/096165

(57) **Abstract**

The present disclosure relates to an artificial intelligence-based mental illness diagnosis system and method using exosome SERS signals.

According to the present disclosure, a mental illness diagnosis system based on artificial intelligence using exosome SERS signals includes a first learning unit configured to cause a mental illness diagnosis algorithm to be learned to classify exosome SERS signals included in input signal maps into 0 and 1 by inputting a first signal map acquired by using exosomes acquired from a normal person and a second signal map acquired by using exosomes acquired from a mental illness patient to the mental illness diagnosis algorithm, a signal acquisition unit configured to drop exosomes acquired from a measurement target person onto a chip including multiple dot arrays and acquire a signal map including multiple exosome SERS signals from the chip, and a diagnosis unit configured to input the acquired signal map to the mental illness diagnosis algorithm for which learning is completed, acquire a signal value of 0 or 1 for each of the exosome SERS signals included in the signal map, and diagnose the measurement target person as the normal person or the mental illness patient by using an average of acquired signal values.

## Description

### Technical Field

The present disclosure relates to an artificial intelligence-based mental illness diagnosis system and method using exosome SERS signals, and more specifically, to a mental illness diagnosis system and method for detecting a SERS signal map for the entire exosome rather than a specific marker and diagnosing and classifying mental illness through an artificial intelligence algorithm learned by using the detected SERS signal map.

### Background Art

Mental illness involves disturbances in thinking, emotion, and behavior, and when the disturbances cause significant distress and interference in daily life, it is diagnosed as a mental illness or mental health disorder.

The effects of mental illness may last for a long time or appear temporarily, and approximately 50% of adults experience mental illness at some points in their lives. The first cause of illness causing mental disorders is depression, and despite being a very common illness, only about 20% of people with mental illness receive professional help.

In general, mental illness is always clearly distinguished from normal behavior, but it is difficult to distinguish between having certain personality and having a personality disorder.

Therefore, in order to secure objectivity in the diagnosis of mental illness, mental illness has been diagnosed based on the criteria presented in the Diagnostic and Statistical Manual of Mental Disorders (DSM) published by the American Psychiatric Association.

However, the Diagnostic and Statistical Manual of Mental Disorders (DSM) is merely a collection of clinical symptoms and has not been verified by laboratory evaluations (radiology, blood tests, neurophysiological tests, and so on). Therefore, research on a psychiatric diagnosis classification system that was verified, as well as clinical medical experience, has been actively conducted recently.

The technology that serves as the background of the present disclosure is disclosed in Japanese Patent Publication No. 2021-112167 (published on August 5, 2021).

### Disclosure of Invention

### Technical Problem

As described above, the present disclosure provides a mental illness diagnosis system and method for detecting a SERS signal map for the entire exosome rather than a specific marker and diagnosing and classifying mental illness through an artificial intelligence algorithm learned by using the detected SERS signal map.

### Solution to Problem

According to an embodiment of the present disclosure, a mental illness diagnosis system based on artificial intelligence using exosome SERS signals includes a first learning unit configured to cause a mental illness diagnosis algorithm to be learned to classify exosome SERS signals included in input signal maps into 0 and 1 by inputting a first signal map acquired by using exosomes acquired from a normal person and a second signal map acquired by using exosomes acquired from a mental illness patient to the mental illness diagnosis algorithm, a signal acquisition unit configured to drop exosomes acquired from a measurement target person onto a chip including multiple dot arrays and acquire a signal map including multiple exosome SERS signals from the chip, and a diagnosis unit configured to input the acquired signal map to the mental illness diagnosis algorithm for which learning is completed, acquire a signal value of 0 or 1 for each of the exosome SERS signals included in the signal map, and diagnose the measurement target person as the normal person or the mental illness patient by using an average of acquired signal values.

The mental illness diagnosis system may further include a classification unit configured to input multiple exosome SERS signals acquired from the measurement target person to multiple mental illness classification algorithms when the measurement target person is diagnosed as the mental illness patient, acquire signal values of 0 or 1 for each of the multiple exosome SERS signals, and classify types of mental illness by using an average of the signal values which are acquired.

The mental illness diagnosis system may further include a SERS signal collection unit configured to acquire the first signal map from the exosomes acquired from the normal person, acquire the second signal map from the exosomes acquired from the mental illness patient, label all of n*m (here, n and m are natural numbers that are equal to or different from each other) exosome SERS signals included in the first signal map as 0, and label all of n*m exosome SERS signals included in the second signal map as 1.

The mental illness diagnosis system may further include a second learning unit configured to input the second signal map acquired from a mental illness patient corresponding to a specific type of mental illness among the mental illness patients, and the second signal map acquired from other mental illness patients excluding the specific type of mental illness to multiple mental illness classification algorithms, and cause the mental illness classification algorithms to be learned to determine whether a signal map which is input corresponds to the specific type of mental illness.

The diagnostic unit may input the n*m exosome SERS signals included in the acquired signal map to the mental illness diagnosis algorithm and output a signal value of 0 or 1 corresponding to each of the n*m exosome SERS signals and may classify as a normal person when an average of output signal values is close to 0, and diagnose as a mental illness patient when the average of the output signal values is close to 1.

The classification unit may input the n*m exosome SERS signals to the multiple mental illness classification algorithms, and the multiple mental illness classification algorithms may output signal values of 0 or 1 for each of the n*m exosome SERS signals that are input, and may compare an average of the signal values with a classification reference value for a specific type of mental illness to determine whether to correspond to the specific type of mental illness.

According to another embodiment of the present disclosure, a mental illness diagnosis method using a mental illness diagnosis system includes a step of causing a mental illness diagnosis algorithm to be learned to classify exosome SERS signals included in input signal maps into 0 and 1 by inputting a first signal map acquired by using exosomes acquired from a normal person and a second signal map acquired by using exosomes acquired from a mental illness patient to the mental illness diagnosis algorithm, a step of dropping exosomes acquired from a measurement target person onto a chip including multiple dot arrays and acquiring a signal map including multiple exosome SERS signals from the chip, and a step of inputting the acquired signal map to the mental illness diagnosis algorithm for which learning is completed, acquiring a signal value of 0 or 1 for each of the exosome SERS signals included in the signal map, and diagnosing the measurement target person as the normal person or the mental illness patient by using an average of acquired signal values.

### Advantageous Effects of Invention

According to the present disclosure, whether there is a mental illness may be diagnosed by inputting an exosome SERS signal map to a mental illness diagnosis algorithm, and furthermore, may specifically diagnose the mental illness by inputting an exosome SERS signal map diagnosed as having a mental illness to multiple mental illness classification algorithms, reanalyzing the exosome SERS signal map, and classifying the type of mental illness.

### Description of Drawings

FIG. 1 is a configuration diagram illustrating a mental illness diagnosis system according to an embodiment of the present disclosure.
FIG. 2 is a flowchart illustrating a mental illness diagnosis method using a mental illness diagnosis system, according to an embodiment of the present disclosure.
FIG. 3 is an example view illustrating step S210 illustrated in FIG. 2.
FIG. 4 is an example diagram illustrating the results of nanoparticle tracking analysis on exosomes of a normal person and exosomes of a depression patient.
FIG. 5 is an example view illustrating a method of labeling exosome SERS signals in step S220 illustrated in FIG. 2.
FIG. 6 is an example view illustrating a method of training a mental illness diagnosis algorithm by using labeled exosome SERS signals in step S220 illustrated in FIG. 2.
FIG. 7 is an example view illustrating the results of a performance evaluation of an artificial intelligence-based mental illness diagnosis method using exosome SERS signals, according to an embodiment of the present disclosure. Best Mode for Carrying out the Invention

Hereinafter, preferred embodiments of the present disclosure will be described in detail with reference to the attached drawings. In this process, thicknesses of lines and sizes of components illustrated in the drawings may be exaggerated for the sake of clarity and convenience of description.

Also, the terms described below are terms defined by considering functions thereof in the present disclosure, and may change depending on the intention or custom of a user or operator. Therefore, definitions of the terms should be made based on the contents throughout the entire specification.

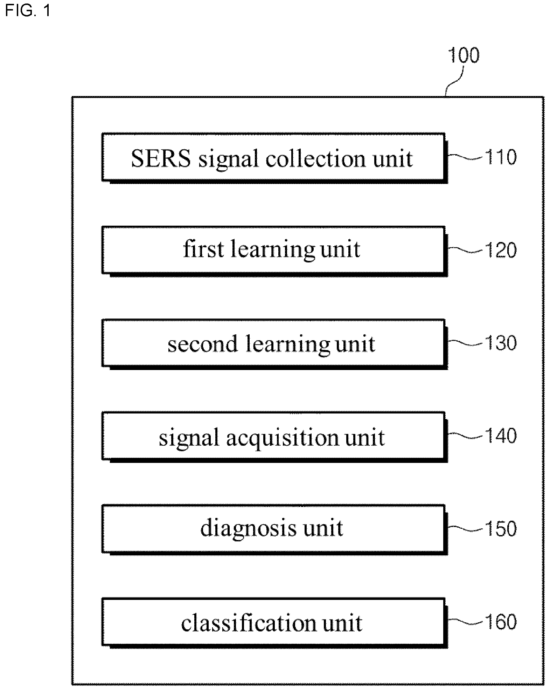

Hereinafter, a mental illness diagnosis system according to an embodiment of the present disclosure is described in more detail with reference to FIG. 1.

FIG. 1 is a configuration diagram illustrating a mental illness diagnosis system according to an embodiment of the present disclosure.

As illustrated in FIG. 1, a mental illness diagnosis system 100 according to an embodiment of the present disclosure includes a SERS signal collection unit 110, a first learning unit 120, a second learning unit 130, a signal acquisition unit 140, a diagnosis unit 150, and a classification unit 160.

First, the SERS signal collection unit 110 performs Raman spectroscopy on exosomes collected from the plasma of a normal person and exosomes collected from the plasma of a mental illness patient to acquire each signal map.

Here, the signal map has a size of n*m according to the number of dot arrays included in a chip. Therefore, the SERS signal collection unit 110 acquires n*m exosome SERS signals according to the size of the signal map.

Hereinafter, for the sake of convenience of description, a signal map acquired from a normal person is referred to as the first signal map, and a signal map acquired from a mental illness patient is referred to as the second signal map.

In addition, the SERS signal collection unit 110 labels n*m exosome SERS signals included in the first signal map as 0, and labels n*m exosome SERS signals included in the second signal map as 1.

The first learning unit 120 constructs a mental illness diagnosis algorithm based on deep learning, and inputs exosome SERS signals labeled as 0 and exosome SERS signals labeled as 1 to the constructed mental illness diagnosis algorithm to train the mental illness diagnosis algorithm. Then, the mental illness diagnosis algorithm outputs a signal value of 0 or 1 in response to the input exosome SERS signal.

The second learning unit 130 constructs a mental illness classification algorithm based on deep learning. In this case, the constructed mental illness classification algorithm includes multiple algorithms corresponding to the types of mental illness. Here, the types of mental illness may include at least one of depression, bipolar disorder, panic disorder, schizophrenia, dementia, and delusional disorder but are not limited thereto and may include various types of mental illnesses.

Next, the second learning unit 130 inputs exosome SERS signals acquired from each mental illness patient to multiple mental illness classification algorithms to cause the multiple mental illness classification algorithms to be learned. Here, the classification algorithm for a specific type of mental illness is learned to determine whether an exosome SESR signal corresponds to a specific type of mental illness by inputting exosome SERS signals acquired from a mental illness patient corresponding to a specific type of mental illness and an exosome SERS signal acquired from a patient with mental illnesses other than the specific type of mental illness. Then, the mental illness classification algorithm outputs a signal value of 0 or 1 for the input exosome SERS signal.

The signal acquisition unit 140 drops exosomes collected from the plasma of a subject of a measurement target person onto a chip, and then performs Raman spectroscopy on the chip to acquire multiple exosome SERS signals.

The diagnosis unit 150 inputs the acquired multiple exosome SERS signals to the mental illness diagnosis algorithm for which learning is completed, and acquires a signal value of 0 or 1 for each exosome SERS signal. Then, the diagnosis unit 150 diagnoses a measurement target person as a normal person or a mentally ill person, by using an average of the acquired signal values.

When the measurement target person is diagnosed to have mental illness, the classification unit 160 inputs the multiple exosome SERS signals respectively to the multiple mental illness classification algorithms for which learning is completed.

Then, the multiple mental illness classification algorithms output signal values of 0 or 1 for the multiple input exosome SERS signals, and the classification unit 160 compares the average of the output signal values with a classification criterion for the corresponding type of mental illness to determine whether to correspond to each type of mental illness.

For example, a depression classification algorithm outputs a signal value of 0 or 1 for the multiple input exosome SERS signals, and compares an average of output signal values with a depression classification criterion to determine whether depression occurs. The bipolar disorder classification algorithm outputs a signal value of 0 or 1 for the multiple input exosome SERS signals, and compares an average of output signal values with a bipolar disorder classification criterion to determine whether bipolar disorder occurs. The panic disorder classification algorithm outputs a signal value of 0 or 1 for the multiple input exosome SERS signals, and compares an average of output signal values with a panic disorder classification criterion to determine whether panic disorder occurs. The schizophrenia classification algorithm outputs a signal value of 0 or 1 for multiple input exosome SERS signals, and compares an average of output signal values with a schizophrenia classification criterion value to determine whether there is schizophrenia. The dementia classification algorithm outputs a signal value of 0 or 1 for multiple input exosome SERS signals, and compares an average of output signal values with a dementia classification criterion value to determine whether there is dementia. Finally, the delusional disorder classification algorithm outputs a signal value of 0 or 1 for multiple input exosome SERS signals, and compares an average of output signal values with a delusional disorder classification criterion value to determine whether there is delusional disorder.

In addition, the classification unit 160 provides information on mental illnesses determined to be positive.

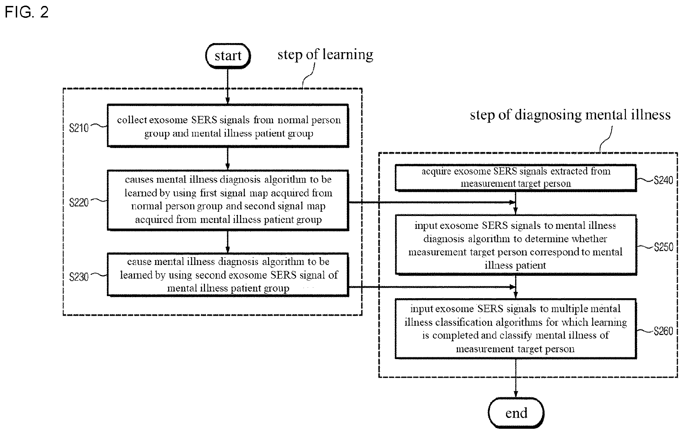

A method of diagnosing mental illness by using a mental illness diagnosis system, according to an embodiment of the present disclosure, is described in more detail with reference to FIG. 2 to FIG. 7.

FIG. 2 is a flowchart illustrating the method of diagnosing mental illness by using the mental illness diagnosis system, according to the embodiment of the present disclosure.

As illustrated in FIG. 2, the method of diagnosing mental illness by using the mental illness diagnosis system, according to an embodiment of the present disclosure, includes a step of learning the mental illness diagnosis algorithm and the mental illness classification algorithm, and a step of diagnosing mental illness by using the mental illness diagnosis algorithm and the mental illness classification algorithm for which learning is completed.

First, in the step of learning the mental illness diagnosis algorithm and the mental illness classification algorithm, the mental illness diagnosis system 100 collects exosome SERS signals from a normal person group and a mental illness patient group (S210).

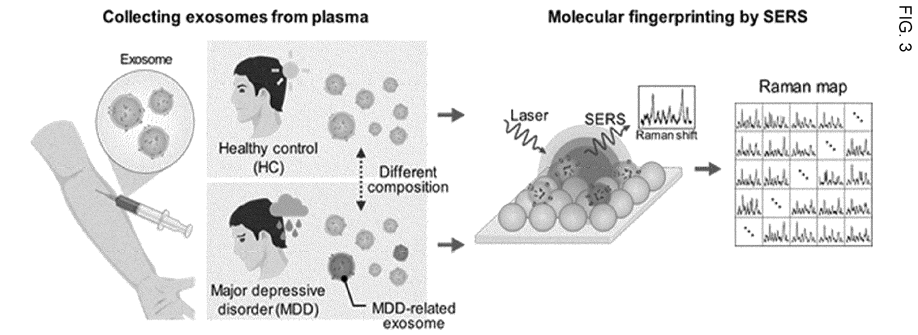

FIG. 3 is an example view illustrating step S210 illustrated in FIG. 2.

As illustrated in FIG. 3, a plasma sample of a normal person is acquired, and exosomes are separated from the acquired plasma by using size exclusion chromatography (SEC). Also, exosomes are separated from a plasma sample of a mental illness patient by using the same method.

In the exosomes separated from the plasma sample of the mental illness patient, exosomes related to mental illness and normal exosomes coexist.

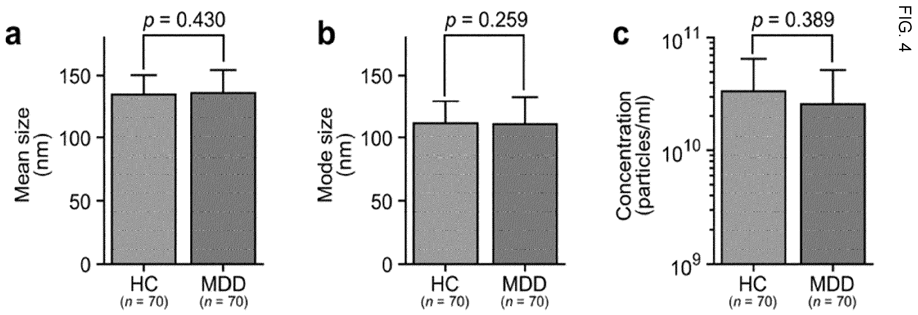

FIG. 4 is an example diagram illustrating the results of nanoparticle tracking analysis on exosomes of a normal person and exosomes of a depression patient.

As illustrated in FIG. 4, according to the results of nanoparticle tracking analysis, it may be seen that there is no significant difference in particle size, mode size, and particle concentration between exosomes of a normal person and exosomes of a depression patient. However, heterogeneity was observed in the signal detected through Raman spectroscopy.

Therefore, according to an embodiment of the present disclosure, exosome SERS signals are detected by using Raman spectroscopy for exosomes separated from plasma samples of a normal person and exosomes separated from a mental illness patient.

In order to describe this in more detail, an exosome solution acquired by being separated from a plasma sample of a normal person and an exosome solution acquired by being separated from a plasma sample of a mental illness patient are dropped onto each Au nanoparticle aggregation array chip and then dried.

Here, the Au nanoparticle aggregation array chip is obtained by precipitating AuNPs (Au nanoparticles) from a colloidal solution, and then coating an APTES-functionalized glass surface with NPs. In order to increase detection throughput and uniformity of the APTES-functionalized glass surface during a signal acquisition process, the Au nanoparticle aggregation array chip includes n*m (here, n and m are natural numbers that are equal to or different from each other) dot arrays, and the exosome SERS signal is measured from each dot.

Next, the SERS signal collection unit 110 collects an exosome SERS signal map including multiple exosome SERS signals corresponding to the dot array by performing Raman spectroscopy on the dried Au nanoparticle aggregation array chip on which an exosome solution is dried.

That is, the SERS signal collection unit 110 collects a first signal map including n*m first exosome SERS signals from the exosome solution of a normal person, and collects a second signal map including n*m second exosome SERS signals from the exosome solution of a mental disorder patient.

The mental disorder diagnosis system 100 according to the embodiment of the present disclosure forms a mental disorder patient group by using patients diagnosed with at least one mental disorder among depression, bipolar disorder, panic disorder, schizophrenia, dementia, and delusional disorder.

When step S210 is completed, the first learning unit 120 causes a mental illness diagnosis algorithm to be learned by using the first signal map acquired from the normal person group and the second signal map acquired from the mental illness patient group (S220).

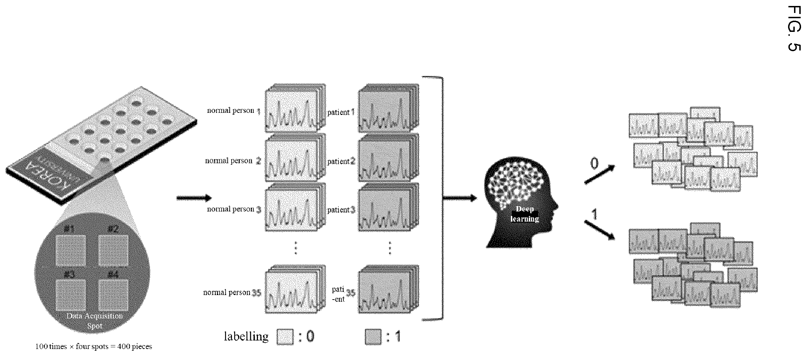

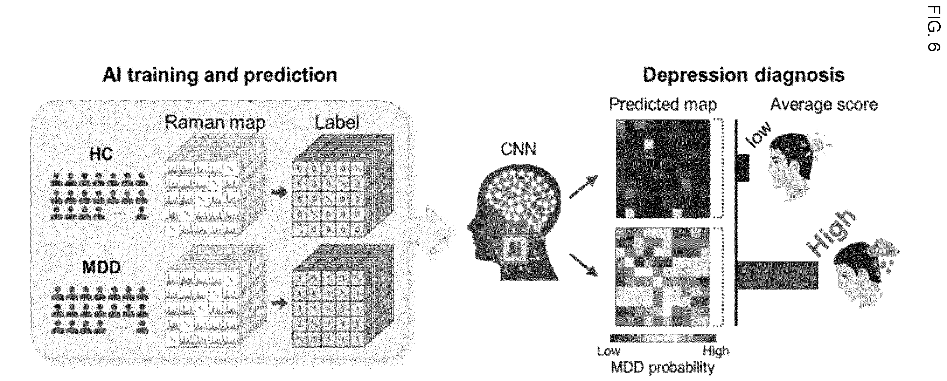

FIG. 5 is an example view illustrating a method of labeling an exosome SERS signal in step S220 illustrated in FIG. 2, and FIG. 6 is an example view illustrating a method of learning a mental illness diagnosis algorithm by using the exosome SERS signal labeled in step S220 illustrated in FIG. 2.

Because the exosome solution of a mental illness patient includes both normal exosomes and mental illness-related exosomes, only one exosome among normal exosomes or mental illness-related exosomes may also exist in the n*m dots arranged on the Au nanoparticle aggregation array chip, or both the normal exosomes and the mental illness-related exosomes may also exist. That is, multiple exosome SERS signals corresponding to the dot array may be output differently.

As illustrated in FIG. 5, the first learning unit 120 according to the embodiment of the present disclosure does not classify the exosome SERS signals according to the presence or absence of the mental illness-related exosomes or normal exosomes, but labels all of the first exosome SERS signals acquired from a normal person as 0, and labels all of the second exosome SERS signals acquired from a mental illness patient as 1.

Then, as illustrated in FIG. 6, the first learning unit 120 randomly extracts learning data and test data from the first exosome SERS signal and the second exosome SERS signal.

Then, the first learning unit 120 causes the mental illness diagnosis algorithm to be learned by using the first exosome SERS signal and the second exosome SERS signal corresponding to the extracted learning data as input data and by using the labeled values as output data.

That is, the mental illness diagnosis algorithm outputs a signal value of 0 or 1 in response to multiple input exosome SERS signals, and first diagnoses whether a mental illness occurs by using an average of output signal values.

Then, the second learning unit 130 causes the mental illness diagnosis algorithm to be learned by using the second exosome SERS signal of the mental illness patient group acquired in step S210 (S230).

The second learning unit 130 constructs multiple mental illness classification algorithms respectively corresponding to depression, bipolar disorder, panic disorder, schizophrenia, dementia, and delusional disorder.

In addition, the second learning unit 130 inputs, to the depression classification algorithm, the second exosome SERS signal acquired from the depression patient group and the second exosome SERS signal acquired from the remaining types of mental illness patient groups excluding the depression patients, and causes the depression classification algorithm to be learned to output a signal value of 0 or 1 in response to the input exosome SERS signal.

Also, the second learning unit 130 inputs, to the panic disorder classification algorithm, the second exosome SERS signal acquired from a bipolar patient group and the second exosome SERS signal acquired from a mental illness patient group other than bipolar patients, and causes the panic disorder classification algorithm to be learned to output a signal value of 0 or 1 in response to the input exosome SERS signal.

The second learning unit 130 also causes the panic disorder classification algorithm, the schizophrenia classification algorithm, the dementia classification algorithm, and the delusional disorder classification algorithm to be learned in the same way.

When learning of the algorithm is completed by using step S210 to step S230, the mental illness diagnosis system 100 diagnoses a measurement target person with a mental illness.

First, the signal acquisition unit 140 acquires exosome SERS signals extracted from a plasma of the measurement target person (S240).

A user collects a plasma from the measurement target person and applies chromatography to the collected plasma to separate exosomes.

Then, the user drops a solution in which exosomes are dissolved onto the Au nanoparticle array chip and dries the solution.

Then, Raman spectroscopy is performed on the Au nanoparticle array chip to acquire n*m (for example, 100) exosome SERS signals corresponding to the dot array.

When step S240 is completed, the diagnosis unit 150 inputs n*m exosome SERS signals to a mental illness diagnosis algorithm to determine whether the measurement target person corresponds to a mental illness patient (S250).

Additionally, the diagnosis unit 150 inputs the n*m exosome SERS signals to a mental illness diagnosis algorithm. Then, the mental illness diagnosis algorithm outputs a signal value of 0 or 1 corresponding to each of the n*m exosome SERS signals.

When an average of output signal values is close to 0, the diagnosis unit 150 diagnoses the measurement target person as a normal person, and when the average of the output signal values is close to 1, the diagnosis unit 150 diagnoses the measurement target person as a mental illness patient.

When the measurement target person is diagnosed as a mental illness patient in step S250, the classification unit 160 inputs the n*m exosome SERS signals to multiple mental illness classification algorithms for which learning is completed and classifies mental illness of the measurement target person (S260).

Additionally, the classification unit 160 inputs the n*m exosome SERS signals respectively to a depression classification algorithm, a bipolar disorder classification algorithm, a panic disorder classification algorithm, a schizophrenia classification algorithm, a dementia classification algorithm, and a delusional disorder classification algorithm.

Then, the depression classification algorithm outputs a signal value of 0 or 1 for each of the input n*m exosome SERS signals, and compares an average of output signal values with a depression classification criterion to determine whether the measurement target person is a depression patient.

The bipolar disorder classification algorithm outputs a signal value of 0 or 1 for each of the input n*m exosome SERS signals, and compares the average of the output signal values with a bipolar disorder classification criterion to determine whether the measurement target person is a bipolar disorder patient.

The panic disorder classification algorithm outputs a signal value of 0 or 1 for each of the input n*m exosome SERS signals, and compares the average of the output signal values with a panic disorder classification criterion to determine whether the measurement target person is a panic disorder patient.

The schizophrenia classification algorithm outputs a signal value of 0 or 1 for each of the input n*m exosome SERS signals, and compares the average of the output signal values with a schizophrenia classification criterion to determine whether the measurement target person is a schizophrenia patient.

The dementia classification algorithm outputs a signal value of 0 or 1 for each of the input n*m exosome SERS signals, and compares the average of the output signal values with a dementia classification criterion value to determine whether the measurement target person is a dementia patient.

Finally, the delusional disorder classification algorithm outputs a signal value of 0 or 1 for each of the input n*m exosome SERS signals, and compares the average of the output signal values with a delusional disorder classification criterion value to determine whether the measurement target person is a delusional disorder patient.

Then, the classification unit 160 provides a mental disorder prediction result by using the results output from the six mental disorder classification algorithms.

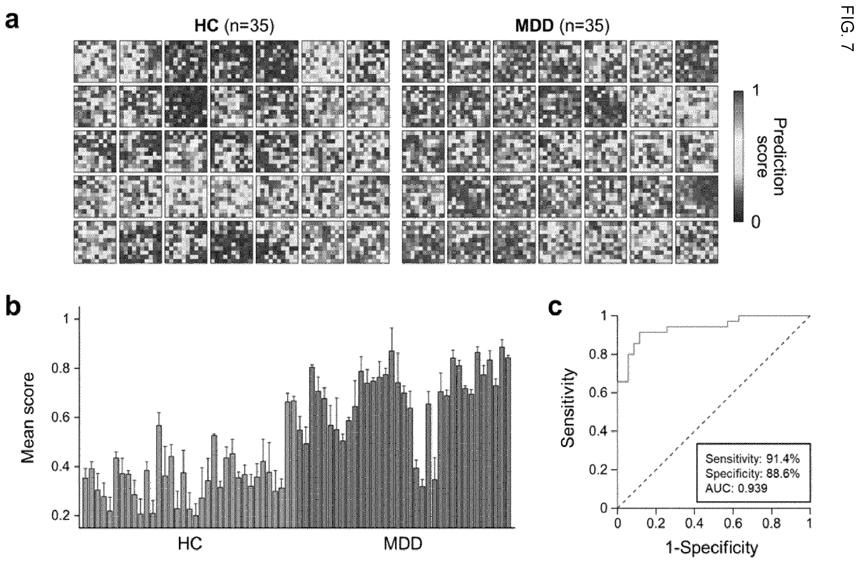

FIG. 7 is an example view illustrating performance evaluation results of an artificial intelligence-based mental disorder diagnosis method using exosome SERS signals, according to an embodiment of the present disclosure.

As illustrated in a of FIG. 7, a heat map acquired from the exosomes of a normal person and a heat map acquired from a depression patient may be easily distinguished with the naked eyes.

In addition, as illustrated in b of FIG. 7, a final diagnostic value is approximately 1.86 times higher in a depression patient group than in a normal person group.

As illustrated in c of FIG. 7, an ROC (Receiver operation characteristic) curve is illustrated to verify effects of the mental illness diagnosis algorithm or mental illness classification algorithm. The closer an area under the ROC curve (AUC: Area under curve) is to 1, the greater the usefulness, and the mental illness diagnosis algorithm or mental illness classification algorithm according to the embodiment of the present disclosure has a value of approximately 0.939, a sensitivity of 91.4 %, and a specificity of approximately 88.6 %. This indicates that the mental illness diagnosis algorithm or mental illness classification algorithm has excellent performance.

In this way, a mental illness diagnosis system according to the present disclosure may diagnose whether there is a mental illness by inputting an exosome SERS signal map to a mental illness diagnosis algorithm, and furthermore, may specifically diagnose the mental illness by inputting an exosome SERS signal map diagnosed as having a mental illness to multiple mental illness classification algorithms, reanalyzing the exosome SERS signal map, and classifying the type of mental illness.

The present disclosure is described with reference to the embodiments illustrated in the drawings, but the embodiments are merely examples, and those skilled in the art will understand that various modifications and equivalent other embodiments may be derived therefrom. Therefore, the true technical protection scope of the present disclosure should be determined by the technical idea of following patent claims.

### <Description of symbols>

100: mental illness diagnosis system
110: SERS signal collection unit
120: first learning unit
130: second learning unit
140: signal acquisition unit
150: diagnosis unit
160: classification unit

## Claims

1. A mental illness diagnosis system based on artificial intelligence using exosome SERS (Surface Enhanced Raman Spectroscopy) signals, the mental illness diagnosis system comprising:
a first learning unit configured to cause a mental illness diagnosis algorithm to be learned to classify exosome SERS signals included in input signal maps into 0 and 1 by inputting a first signal map acquired by using exosomes acquired from a normal person and a second signal map acquired by using exosomes acquired from a mental illness patient to the mental illness diagnosis algorithm;
a signal acquisition unit configured to drop exosomes acquired from a measurement target person onto a chip including multiple dot arrays and acquire a signal map including multiple exosome SERS signals from the chip; and
a diagnosis unit configured to input the acquired signal map to the mental illness diagnosis algorithm for which learning is completed, acquire a signal value of 0 or 1 for each of the exosome SERS signals included in the signal map, and diagnose the measurement target person as the normal person or the mental illness patient by using an average of acquired signal values.

2. The mental illness diagnosis system of claim 1, further comprising:
a classification unit configured to input multiple exosome SERS signals acquired from the measurement target person to multiple mental illness classification algorithms when the measurement target person is diagnosed as the mental illness patient, acquire signal values of 0 or 1 for each of the multiple exosome SERS signals, and classify types of mental illness by using an average of the signal values which are acquired.

3. The mental illness diagnosis system of claim 2, further comprising:
a SERS signal collection unit configured to acquire the first signal map from the exosomes acquired from the normal person, acquire the second signal map from the exosomes acquired from the mental illness patient, label all of n*m (here, n and m are natural numbers that are equal to or different from each other) exosome SERS signals included in the first signal map as 0, and label all of n*m exosome SERS signals included in the second signal map as 1.

4. The mental illness diagnosis system of claim 3, further comprising:
a second learning unit configured to input the second signal map acquired from a mental illness patient corresponding to a specific type of mental illness among the mental illness patients, and the second signal map acquired from other mental illness patients excluding the specific type of mental illness to multiple mental illness classification algorithms, and cause the mental illness classification algorithms to be learned to determine whether a signal map which is input corresponds to the specific type of mental illness.

5. The mental illness diagnosis system of claim 3, wherein
the diagnostic unit inputs the n*m exosome SERS signals included in the acquired signal map to the mental illness diagnosis algorithm and outputs a signal value of 0 or 1 corresponding to each of the n*m exosome SERS signals, and classifies as a normal person when an average of output signal values is close to 0, and diagnoses as a mental illness patient when the average of the output signal values is close to 1.

6. The mental illness diagnosis system of claim 4, wherein
the classification unit inputs the n*m exosome SERS signals to the multiple mental illness classification algorithms, and the multiple mental illness classification algorithms output signal values of 0 or 1 for each of the n*m exosome SERS signals that are input, and compares an average of the signal values with a classification reference value for a specific type of mental illness to determine whether to correspond to the specific type of mental illness.

7. A mental illness diagnosis method using a mental illness diagnosis system, the mental illness diagnosis method comprising:
a step of causing a mental illness diagnosis algorithm to be learned to classify exosome SERS signals included in input signal maps into 0 and 1 by inputting a first signal map acquired by using exosomes acquired from a normal person and a second signal map acquired by using exosomes acquired from a mental illness patient to the mental illness diagnosis algorithm;
a step of dropping exosomes acquired from a measurement target person onto a chip including multiple dot arrays and acquiring a signal map including multiple exosome SERS signals from the chip; and
a step of inputting the acquired signal map to the mental illness diagnosis algorithm for which learning is completed, acquiring a signal value of 0 or 1 for each of the exosome SERS signals included in the signal map, and diagnosing the measurement target person as the normal person or the mental illness patient by using an average of acquired signal values.
